# EUROPÄISCHE PATENTSCHRIFT

(11) **EP 1 406 485 B1**
(45) Veröffentlichungstag und Bekanntmachung des Hinweises auf die Patenterteilung: **14.09.2005**
(21) Anmeldenummer: 02762314.9
(22) Anmeldetag: 02.07.2002
(51) Int. Cl.: A01J 11/10, B04B 11/02, A23C 9/15, G01N 33/06

(54) **VERFAHREN UND VORRICHTUNG ZUR ÜBERWACHUNG, STEUERUNG UND/ODER REGELUNG EINER ZENTRIFUGE**
METHOD FOR MONITORING, CONTROL AND/OR REGULATION OF A CENTRIFUGE
PROCEDE ET DISPOSITIF DE CONTROLE, COMMANDE ET/OU REGULATION D'UNE CENTRIFUGEUSE

(30) Priorität: 18.07.2001 DE 10135073
(43) Veröffentlichungstag der Anmeldung: 14.04.2004
(73) Patentinhaber: Westfalia Separator AG, 59302 Oelde (DE)
(72) Erfinder: ZETTIER, Karl-Heinz, D-59302 Oelde (DE)
(74) Vertreter: Specht, Peter
(86) Internationale Anmeldenummer: PCT/EP2002/007258
(87) Internationale Veröffentlichungsnummer: WO 2003/007700

(56) Entgegenhaltungen:
- WO-A-00/49388
- US-A- 2 542 456
- US-A- 4 981 610

## Beschreibung

Die Erfindung betrifft ein Verfahren zur Steuerung und/oder Regelung einer Zentrifuge bei der zentrifugalen Gewinnung eines Milchproduktes, bei welchem Milch unter Minimierung des Fettgehaltes in der Magermilch in Magermilch und Rahm getrennt wird.

Es ist bekannt, mit Hilfe von Zentrifugen Milch in Magermilch und Rahm aufzutrennen. Hierzu werden im allgemeinen Tellerseparatoren eingesetzt.

Bei der Aufteilung in Magermilch und Rahm ist es ein besonderes Ziel der zentrifugalen Trennung, den Fettgehalt in der Magermilch möglichst zu minimieren, um die wirtschaftliche Ausbeute des Verfahrens so optimal wie möglich zu gestalten.

Leider ist die Bestimmung des Fettgehaltes von Rahm oder Magermilch relativ problematisch, da die Magermilch oder der Rahm ein weiße Färbung aufweisen, so daß Verfahren zur Bestimmung der Lichtdurchlässigkeit der Magermilch - wie nach der Erfindung vorgesehen - zur Bestimmung des Fettgehaltes nicht zum Einsatz kommen können sondern nur andere aufwendige (Labor-)Verfahren wie das von Röse-Gottlieb und das von Mojonier mit Genauigkeiten bzw. einer Reproduzierbarkeit von 0,03% und 0,015%. Aus der WO 00/49388 ist es hierzu bekannt, einer Milchprobe einen die Lichtdurchlässigkeit erhöhenden Stoff zuzusetzen und dann eine Durchleuchtung der Milchprobe vorzunehmen.

Aus der US 2,542,456 ist es bekannt, einem der beiden Abläufe einer Zentrifuge zur Gewinnung eines Milchproduktes eine kleinere, nachgeschaltete Zentrifuge aus transparentem Material nachzuordnen, und mit dieser bei der Gewinnung von Rahm die Rahmprobe und bei der Herstellung standardisierter Milch die standardisierte Milch zu durchleuchten. Mittels eines photosensitiven Elementes werden dabei die Fettgehalte ermittelt. Dieses Verfahren ist allerdings nicht mehr an modernen Separatoren zur Trennung von Milch in Rahm und Magermilch einsetzbar, bei denen der Restfettgehalt in der Magermilch auf nur wenige zehntel Prozent Fettgehalt zu minimieren ist, da in diesem Fall eine zuverlässige Fettgehaltbestimmung in der Rahmphase nicht genau genug ist.

Die Erfindung hat vor diesem Hintergrund die Aufgabe, die Restfettgehaltbestimmung bei der zentrifugalen Trennung von Milch in Magermilch und Rahm zu vereinfachen

Die Erfindung löst diese Aufgabe in Hinsicht auf das Verfahren durch den Gegenstand des Anspruches 1 und in Hinsicht auf die Vorrichtung durch den Gegenstand des Anspruches 6.

Insbesondere wird die Lichtdurchlässigkeit der Flüssigkeitsphase aus Magermilch dadurch erhöht, daß der Milchprobe ein den pH-Wert erhöhender Stoffeine geeignete alkalische Lösung - zugesetzt wird. Daraufhin kann die Lichtdurchlässigkeit mittels einfachen Durchleuchtens der Milchprobe mit einer Lichtquelle und einer der Milchprobe zugeordneten Fotozelle ermittelt werden.

Die Erfindung hat den besonderen Vorteil, daß es durch Zusatz des den pH-Wert erhöhenden Stoffes auf einfache Weise möglich wird, die Lichtdurchlässigkeit der weißen Probe so stark zu erhöhen, daß es überraschend möglich wird, ein optisches Verfahren zur Bestimmung des Fettgehaltes der Magermilch einzusetzen. Dabei wird die Fettgehaltbestimmung automatisiert mit einem Rechner in Intervallen durchgeführt werden. Wird der Rechner wiederum mit den Steuereingängen der eigentlichen Zentrifugensteuerung verbunden oder wird sogar der Rechner zur Steuerung der Zentrifuge benutzt, wird es möglich, die Informationen nicht nur zur Überwachung der Einstellung der Zentrifuge, sondern auch zu deren Steuerung und/oder auch zur Regelung in Abhängigkeit von der Fettgehaltbestimmung zu nutzen.

Vorteilhaft wird die Lichtdurchlässigkeit der Flüssigkeitsphase, insbesondere der Magermilch, dadurch erhöht, dass der den pH-Wert erhöhende Stoff derart bemessen und zugesetzt wird, daß der pH-Wert der Milchprobe auf 11 - 14, vorzugsweise 12 - 13 und besonders zweckmäßig auf 13 erhöht wird.

Vorteilhafte Ausgestaltungen sind den übrigen Unteransprüchen zu entnehmen.

Nachfolgend werden Ausführungsbeispiele anhand der Zeichnungen näher beschrieben.

Es zeigt:
- Figur 1: eine Vorrichtung zur Durchführung des Verfahrens zur Überwachung, Steuerung und/oder Regelung einer Zentrifuge,
- Figur 2: ein Diagramm zur Veranschaulichung der Reproduzierbarkeit der Mess-Ergebnisse sowie
- Figur 3: ein Diagramm zur Veranschaulichung des Verhältnisses vom Fettgehalt zum Anzeigewert der Fotozelle.

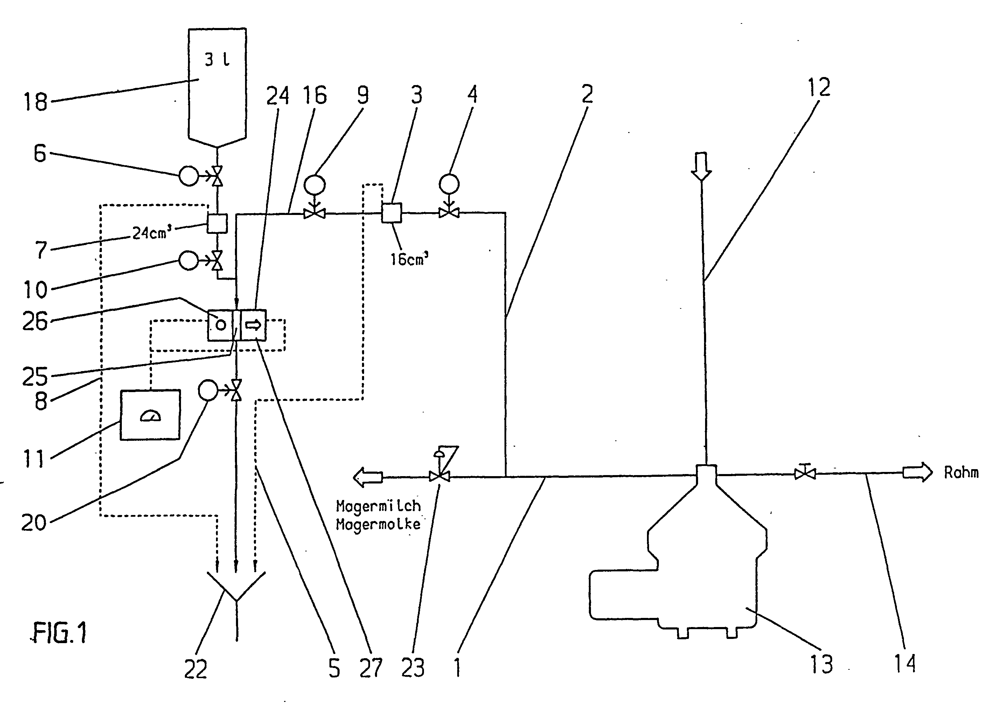

Figur 1 zeigt eine als Tellerseparator 13 ausgebildete Zentrifuge mit einem Zulauf 12 sowie einem Ablauf 14 für Rahm und einem Ablauf 1 für Magermilch. Nach der zentrifugalen Auftrennung des zulaufenden Milchproduktes in Rahm und Magermilch ist es möglich, über eine in den Ablauf 1 der Zentrifuge geschaltete Probeentnahmeleitung 2 dem Ablauf 1 der Zentrifuge Magermilch zu entnehmen und diese in einen ersten Vorratsbehälter 3 zu leiten. Die Entnahme erfolgt vor dem der Zentrifuge zugeordneten bzw. hier nachgeschalteten Regel- bzw.- Konstantdruckventil 23, um die Milchprobe aus einem weitgehend schaumfreien Bereich zu entnehmen.

Dem Vorratsbehälter 3 ist ein Ventil 4 vorgeschaltet, welches bei der Befüllung des Vorratsbehälters 3 solange geöffnet wird, bis Magermilch über ein dem Vorratsbehälter zugeordnetes Entlüftungsrohr 5 austritt, welches in den Auffangbehälter 22 mündet. Die Größe des ersten Vorratsbehälters 3 bestimmt das Volumen, welches zur Messung erforderlich ist bzw. genutzt wird.

Dem ersten Vorratsbehälter 3 ist ein weiteres Ventil 9 zum Ableiten der Magermilchprobe aus dem Vorratsbehälter 3 nachgeschaltet. Das Ventil 9 am Ablauf des Vorratsbehälters 3 ist über eine Leitung 16 mit einer Meßzelle 24 verbunden, welche aus einem Probenaufnahmebehälter 25, einer Lichtquelle 26 und einer Photozelle 27 besteht, wobei die Meßzelle mit einer Auswertungs- und/oder Anzeigeeinrichtung 11 (z.B. ein Rechner, der auch die Messungen steuert) verbunden sind.

Der Meßzelle 24 ist ferner aus einem zweiten Vorratsbehälter 18 eine Flüssigkeit zur Erhöhung des pH-Wertes der aus dem Vorratsbehälter 3 abgeleiteten Milchprobe vorgeschaltet. Aus dem Vorratsbehälter 18 ist die den pH-Wert erhöhende Flüssigkeit bzw. der den pH-Wert erhöhende Stoff über ein automatisches Ventil 6 in einen dritten Vorratsbehälter 7 leitbar. Wiederum bestimmt die Größe des dritten Vorratsbehälters 7 das benötigte Volumen. Der Vorratsbehälter 7 ist gefüllt, wenn aus einer dem Vorratsbehälter 7 zugeordneten Überlaufbohrung 8, welche in den Auffangbehälter 22 führt, Flüssigkeit austritt.

Sobald die beiden Vorratsbehälter 7 und 3 gefüllt sind, werden die den beiden Vorratsbehältern 7 und 3 nachgeschalteten Ventile 9 und 10 geöffnet. Dies bewirkt, daß die in den Vorratsbehältern 3 und 7 enthaltenen Flüssigkeiten über die Ventile 9 und 10 in den Probenaufnahmebehälter 25 strömen und sich dort vermischen. Durch die Zugabe des Stoffes aus dem dritten Vorratsbehälter 7 wird der pH-Wert der Magermilch so angehoben, daß die Struktur des in der Magermilch befindlichen Eiweißes sich so ändert, daß eine Lichtdurchlässigkeit der Milchprobe erreicht wird.

Vorteilhaft beträgt daß Mengenverhältnis aus den Vorratsbehältern 2 und 7 2:3, wenn eine geeignete alkalische Lösung zur pH-Wert-Erhöhung eingesetzt wird.

Anhand dieser Lichtdurchlässigkeit wird jetzt der Restfettgehalt bestimmbar. Hierzu wird die Lichtdurchlässigkeit ermittelt, was mittels eines Bestrahlens der Milchprobe mit der Lichtquelle 26 und der relativ zur Lichtquelle hinter der Meßzelle angeordneten Photozelle 27 erfolgt (hier nicht dargestellt).

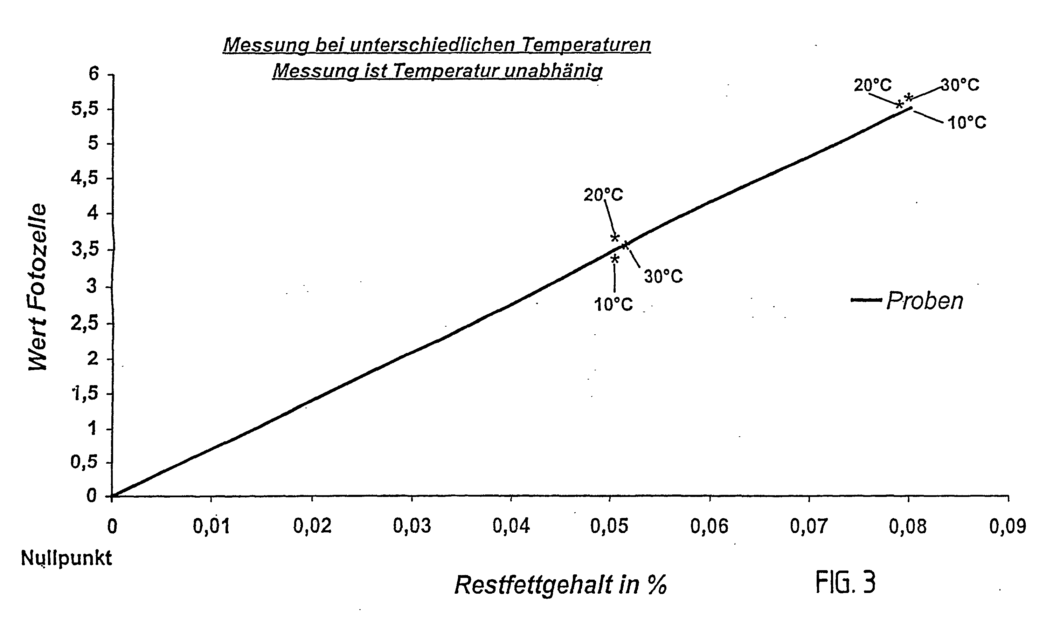

Empirisch kann eine entsprechende Tabelle, welche die Abhängigkeit der Lichtdurchlässigkeit von Restfettgehalt angibt, ermittelt und dann in einem Rechner abgespeichert werden, so daß nach der Messung der Lichtdurchlässigkeit der Milchprobe die Trübung und damit der Fettgehalt anhand eines Vergleiches mit der abgespeicherten Tabelle bestimmt werden. Durch eine entsprechende Justierung ist es sogar denkbar, der Auswertungs- und/oder Anzeigeeinrichtung 11 direkt mit einer Skala zu versehen, welche den Restfettgehalt anzeigt, wenn dort eine zur Lichtdurchlässigkeit proportionale Anzeige realisiert wird. Auf diese Weise wird die Auswertung der Fettgehaltbestimmung weiter vereinfacht. Dass diese Anzeige realisierbar ist, zeigt Fig. 3, welche die Temperaturunabhängigkeit der Messungen und die Proportionalität zwischen der Fotozellenanzeige und dem Restfettgehalt veranschaulicht.

Schwache, fast kristallklare Magermilchproben weisen beispielsweise einen Restgehalt von ca. 0,05 Prozent Fett auf. Eine starke Trübung läßt dagegen auf einen Restfettgehalt von ca. 0,15 Prozent schließen.

Nach der Messung wird die Milchprobe aus der Meßzelle 24 beispielsweise über ein Ventil 20 in den Auffangbehälter 22 abgeleitet.

Anhand des Meßergebnisses kann die Einstellung der Zentrifuge bei Abweichungen vom Sollwert entweder manuell oder aber automatisch - z.B. mittels eines an die Meßzelle und die Tellerzentrifuge 13 angeschlossenen Rechners (hier nicht dargestellt) - geändert werden.

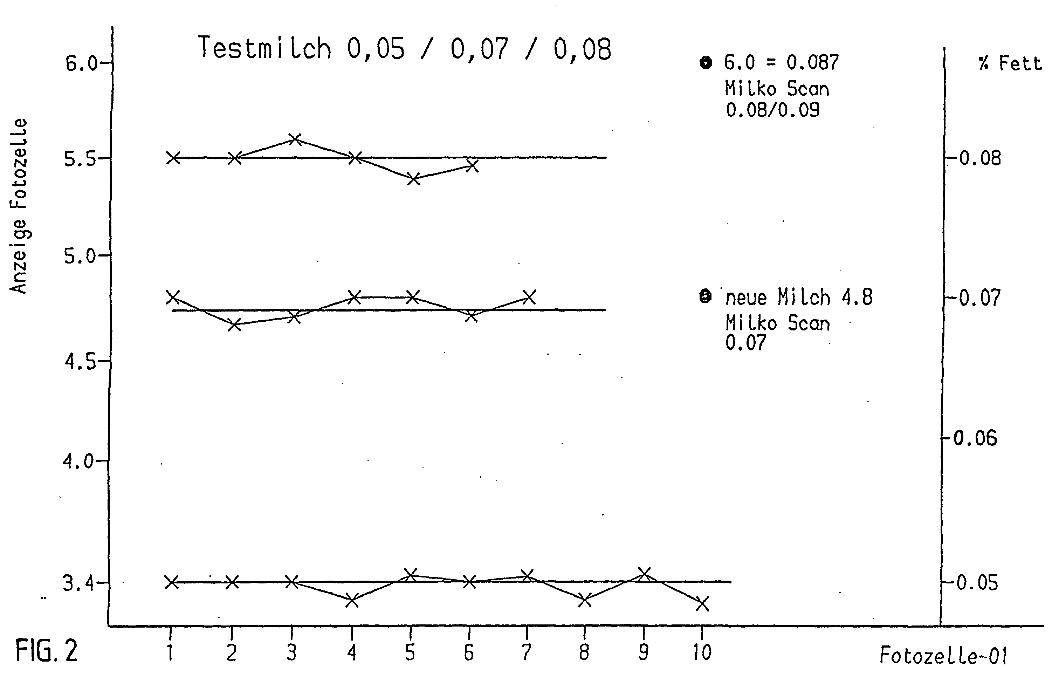

Figur 2 verdeutlicht, daß eine Reproduzierbarkeit im Bereich von 1/1000 möglich ist. Diese Genauigkeit macht es möglich, die neue Meßmethode zum Steuerung und/oder Regeln von Separatoren einzusetzen. Bei einer Verschlechterung des Entrahmungswertes können z.B. eine volle Leerung oder eine CIP durchgeführt werden. Realistisch erscheint sogar möglicherweise eine Reproduzierbarkeit von weniger als 0,005%.

### Bezugszeichenliste

- 1: Ablauf
- 2: Probeentnahmeleitung
- 3: Vorratsbehälter
- 4: Ventil
- 5: Entlüftungsrohr
- 6: Ventil
- 7: Vorratsbehälter
- 8: Überlaufrohr
- 9: Ventil
- 10: Ventil
- 11: Auswertungs- und/oder Anzeigeeinrichtung
- 12: Zulauf
- 13: Tellerseparator
- 14: Ablauf
- 16: Leitung
- 18: Vorratsbehälter
- 20: Ventil
- 22: Auffangbehälter
- 23: Konstantdruckventil
- 24: Meßzelle
- 25: Probenaufnahmebehälter
- 26: Lichtquelle
- 27: Photozelle

## Patentansprüche

1. Verfahren zur Steuerung und/oder Regelung einer Zentrifuge bei der zentrifugalen Gewinnung eines Milchproduktes, bei welchem Milch unter einer Minimierung des Fettgehaltes in der Magermilch in Magermilch und Rahm getrennt wird, mit folgenden Schritten:
a) an einem Ablauf (1) der Zentrifuge (13) wird der ablaufenden Flüssigkeitsphase aus Magermilch eine Milchprobe aus Magermilch entnommen,
b) der Milchprobe aus Magermilch wird ein die Lichtdurchlässigkeit erhöhender Stoff zugesetzt,
c) daraufhin wird die Lichtdurchlässigkeit der Milchprobe aus Magermilch ermittelt und daraus der Fettgehalt automatisiert mit einem Rechner in Intervallen bestimmt, und
d) in Abhängigkeit der Fettgehaltbestimmung wird die Einstellung der Zentrifuge (13) gesteuert und/oder geregelt,
e) wobei die Lichtdurchlässigkeit der Milchprobe aus Magermilch mittels eines Durchleuchtens der Milchprobe mit einer Lichtquelle (26) und eines Messens der Lichtintensität mit einer der Milchprobe zugeordneten Fotozelle (27) ermittelt wird.

2. Verfahren nach Anspruch 1, **dadurch gekennzeichnet, dass** die Lichtdurchlässigkeit der Milchprobe, aus Magermilch, **dadurch** erhöht wird, dass der Milchprobe ein den pH-Wert erhöhender Stoff zugesetzt wird.

3. Verfahren nach Anspruch 2, **dadurch gekennzeichnet, dass** die Lichtdurchlässigkeit der Flüssigkeitsphase aus Magermilch **dadurch** erhöht wird, dass der den pH-Wert erhöhende Stoff derart bemessen und zugesetzt wird, daß der pH-Wert der Milchprobe auf 11 - 14 erhöht wird.

4. Verfahren nach Anspruch 3, **dadurch gekennzeichnet, dass** die Lichtdurchlässigkeit der Flüssigkeitsphase aus Magermilch, **dadurch** erhöht wird, dass der den pH-Wert erhöhende Stoff derart bemessen und zugesetzt wird, daß der pH-Wert der Milchprobe auf 12 - 13 erhöht wird.

5. Verfahren nach Anspruch 4, **dadurch gekennzeichnet, dass** die Lichtdurchlässigkeit der Flüssigkeitsphase aus Magermilch **dadurch** erhöht wird, dass der den pH-Wert erhöhende Stoff derart bemessen und zugesetzt wird, daß der pH-Wert der Milchprobe auf 13 erhöht wird.

6. Vorrichtung zur Durchführung des Verfahrens nach einem der vorstehenden Ansprüche, mit
a) einer Einrichtung mit Probeentnahmeleitung (2) und Vorratsbehälter (3) zur Entnahme einer Milchprobe der Magermilch aus dem Ablauf der als Tellerseparator (13) ausgebildeten Zentrifuge,
b) einer Einrichtung mit Ventil (6, 10) zum Zusetzen eines die Lichtdurchlässigkeit erhöhender Stoff zur Milchprobe aus Magermilch, und
f) einer Einrichtung mit Messzelle (24), Probenaufnahmebehälter (25), Lichtquelle (26), Photozelle(27) zur Ermittlung der Lichtdurchlässigkeit der Milchprobe aus Magermilch und zur Bestimmung des Fettgehaltes, die einen Rechner aufweist, mit dem daraufhin die Fettgehalt der Milchprobe aus Magermilch automatisiert in Intervallen bestimmbar ist, und
c) eine Einrichtung zur Steuerung und/oder Regelung der Einstellung der Zentrifuge in Abhängigkeit vom ermittelten Fettgehalt zugeordnet ist.

7. Vorrichtung nach Anspruch 6, **dadurch gekennzeichnet, daß** dem Ablauf (1) des Tellerseparators eine Probeentnahmeleitung (2) zugeordnet ist, welche vorzugsweise über ein Ventil (4) in einen ersten Vorratsbehälter (3) mündet.

8. Vorrichtung nach Anspruch 6 oder 7, **dadurch gekennzeichnet, daß** die Probeentnahmeleitung (2) vor ein Konstantdruckventil (23) der Zentrifuge geschaltet ist.

9. Vorrichtung nach Anspruch 6, 7 oder 8, **dadurch gekennzeichnet, daß** der Vorratsbehälter (3) für die Milchprobe aus Magermilch über ein Ventil (9) mit einer optischen Messzelle (24) verbunden ist.

10. Vorrichtung nach Anspruch 9, **dadurch gekennzeichnet, daß** der Messzelle (24) femer mindestens ein weiterer Vorratsbehälter (18) zur Aufnahme einer Flüssigkeit bzw. eines Stoffes zur Erhöhung des pH-Wertes der Milchprobe aus Magermilch vorgeschaltet ist.

11. Vorrichtung nach einem der vorstehenden Ansprüche 6-10, **dadurch gekennzeichnet, daß** die Messzelle (24) einen Probenaufnahmebehälter (25), eine Lichtquelle (26) und eine Photozelle (27) aufweist.

12. Vorrichtung nach einem der vorstehenden Ansprüche 6-11, **dadurch gekennzeichnet, daß** der Messzelle (24) eine Auswertungs- und/oder Anzeigeeinrichtung (11) zugeordnet ist.

13. Vorrichtung nach Anspruch 12, **dadurch gekennzeichnet, daß** die Auswertungs- und/oder Anzeigeeinrichtung (11) mit einer Skala versehen ist, welche direkt den Restfettgehalt der Milchprobe anzeigt.

14. Vorrichtung nach einem der vorstehenden Ansprüche 6-13, **gekennzeichnet durch** eine Ausbildung als separate Messeinheit, welche der Zentrifuge zuzuordnen ist.

## Claims

1. Method for the open-loop control and/or closed-loop control of a centrifuge in the centrifugal production of a milk product in which milk is separated into skimmed milk and cream, with the fat content in the skimmed milk being minimized, having the following steps:
a) a milk sample of skimmed milk is taken off from the effluent liquid phase of skimmed milk at an outlet (1) of the centrifuge (13),
b) a substance increasing the light transmittance is added to the milk sample of skimmed milk,
c) the light transmittance of the milk sample of skimmed milk is thereupon determined and the fat content is determined therefrom in an automated manner at intervals using a computer, and
d) the setting of the centrifuge (13) is subjected to open-loop control and/or closed-loop control as a function of the fat content determination,
e) the light transmittance of the milk sample of skimmed milk being determined by means of transillumination of the milk sample by a light source (26) and measurement of the light intensity by a photocell (27) assigned to the milk sample.

2. Method according to Claim 1, **characterized in that** the light transmittance of the milk sample of skimmed milk is increased by adding a pH-increasing substance to the milk sample.

3. Method according to Claim 2, **characterized in that** the light transmittance of the liquid phase of skimmed milk is increased by quantifying and adding the pH-increasing substance in such a manner that the pH of the milk sample is increased to 11-14.

4. Method according to Claim 3, **characterized in that** the light transmittance of the liquid phase of skimmed milk is increased by quantifying and adding the pH-increasing substance in such a manner that the pH of the milk sample is increased to 12-13.

5. Method according to Claim 4, **characterized in that** the light transmittance of the liquid phase of skimmed milk is increased by quantifying and adding the pH-increasing substance in such a manner that the pH of the milk sample is increased to 13.

6. Apparatus for carrying out the method according to one of the preceding claims, having
a) a device having a sample withdrawal line (2) and storage container (3) for withdrawing a milk sample of skimmed milk from the outlet of the centrifuge which is constructed as a disc separator (13),
b) a device having a valve (6; 10) for adding a substance increasing the light transmittance to the milk sample of skimmed milk, and
f) a device having a measuring cell (24), sample reception container (25), light source (26), photocell (27) for determining the light transmittance of the milk sample of skimmed milk and for determining the fat content, which device has a computer using which the fat content of the milk sample of skimmed milk can thereupon be determined in an automated manner at intervals, and
c) a device for the open-loop control and/or closed-loop control of the setting of the centrifuge as a function of the fat content which is determined.

7. Apparatus according to Claim 6, **characterized in that** a sample withdrawal line (2) is assigned to the outlet (1) of the disc separator, which sample withdrawal line opens out, preferably via a valve (4), into a first storage container (3).

8. Apparatus according to Claim 6 or 7, **characterized in that** the sample withdrawal line (2) is connected to the centrifuge upstream of a constant-pressure valve (23).

9. Apparatus according to Claim 6, 7 or 8, **characterized in that** the storage container (3) for the milk sample of skimmed milk is connected via a valve (9) to an optical measuring cell (24).

10. Apparatus according to Claim 9, **characterized in that** at least one further storage container (18) for receiving a liquid or a substance for increasing the pH of the milk sample of skimmed milk is connected upstream of the measuring cell (24).

11. Apparatus according to one of the preceding Claims 6-10, **characterized in that** the measuring cell (24) has a sample reception container (25), a light source (26) and a photocell (27).

12. Apparatus according to one of the preceding Claims 6-11, **characterized in that** a data analysis and/or display device (11) is assigned to the measuring cell (24).

13. Apparatus according to Claim 12, **characterized in that** the data analysis and/or display device (11) is provided with a scale which directly displays the residual fat content of the milk sample.

14. Apparatus according to one of the preceding Claims 6-13, **characterized by** a construction as a separate measuring unit which is to be assigned to the centrifuge.

## Revendications

1. Procédé pour la commande et/ou pour la régulation d'une centrifugeuse lors de la production par centrifugation d'un produit laitier, dans lequel du lait est séparé en lait écrémé et en crème tout en minimisant la teneur en matières grasses dans le lait écrémé, avec les étapes suivantes :
a) sur un écoulement (1) de la centrifugeuse (13), un échantillon de lait composé de lait écrémé est prélevé dans la phase liquide se composant de lait écrémé qui s'écoule,
b) une substance augmentant la perméabilité à la lumière est ajoutée à l'échantillon de lait composé de lait écrémé,
c) ensuite la perméabilité à la lumière de l'échantillon de lait composé de lait écrémé est déterminée et la teneur en matières grasses en est déduite par intervalles et de manière automatisée à l'aide d'un calculateur et
d) le réglage de la centrifugeuse (13) est commandé et/ou est régulé en fonction de la détermination de la teneur en matières grasses,
e) la perméabilité à la lumière de l'échantillon de lait composé de lait écrémé étant déterminée au moyen d'un examen de l'échantillon de lait par transparence à la lumière à l'aide d'une source lumineuse (26) et au moyen d'une mesure de l'intensité de la lumière à l'aide d'une cellule photoélectrique (27) associée à l'échantillon de lait.

2. Procédé selon la revendication 1, **caractérisé en ce que** la perméabilité à la lumière de l'échantillon de lait composé de lait écrémé est augmentée par le fait qu'une substance augmentant le pH est rajoutée à l'échantillon de lait.

3. Procédé selon la revendication 2, **caractérisé en ce que** la perméabilité à la lumière de la phase liquide se composant de lait écrémé est augmentée par le fait que la substance augmentant le pH est déterminée et est rajoutée de telle façon que le pH de l'échantillon de lait soit augmenté à 11 - 14.

4. Procédé selon la revendication 3, **caractérisé en ce que** la perméabilité à la lumière de la phase liquide se composant de lait écrémé est augmentée par le fait que la substance augmentant le pH est déterminée et est rajoutée de telle façon que le pH de l'échantillon de lait soit augmenté à 12 - 13.

5. Procédé selon la revendication 4, **caractérisé en ce que** la perméabilité à la lumière de la phase liquide se composant de lait écrémé est augmentée par le fait que la substance augmentant le pH est déterminée et est rajoutée de telle façon que le pH de l'échantillon de lait soit augmenté à 13.

6. Dispositif pour la mise en oeuvre du procédé selon l'une des revendications précédentes
a) avec un équipement disposant d'une conduite de prélèvement d'échantillons (2) et d'un réservoir de stockage (3) pour le prélèvement d'un échantillon de lait écrémé dans l'écoulement de la centrifugeuse configurée comme séparateur à disques (13),
b) avec un équipement disposant d'une vanne (6 ; 10) pour l'adjonction à l'échantillon de lait composé de lait écrémé d'une substance augmentant la perméabilité à la lumière,
f) avec un équipement disposant d'une cellule de mesure (24), d'un réservoir de collecte des échantillons (25), d'une source lumineuse (26) et d'une cellule photo-électrique (27) pour déterminer la perméabilité à la lumière de l'échantillon de lait composé de lait écrémé et pour définir la teneur en matières grasses, lequel équipement comprend un calculateur avec lequel il est ensuite possible de déterminer par intervalles et de manière automatisée la teneur en matières grasses, et
c) avec un équipement pour la commande et/ou pour la régulation de l'ajustement de la centrifugeuse en fonction de la teneur en matières grasses qui a été déterminée.

7. Dispositif selon la revendication 6, **caractérisé en ce qu'**à l'écoulement (1) du séparateur à disques est associée une conduite de prélèvement d'échantillons (2) qui aboutit, de préférence par l'intermédiaire d'une vanne (4), dans un premier réservoir de stockage (3).

8. Dispositif selon la revendication 6 ou 7, **caractérisé en ce que** la conduite de prélèvement d'échantillons (2) est placée devant une vanne à pression constante (23) de la centrifugeuse.

9. Dispositif selon la revendication 6, 7 ou 8, **caractérisé en ce que** le réservoir de stockage (3) pour l'échantillon de lait composé de lait écrémé est relié par l'intermédiaire d'une vanne (9) avec une cellule optique de mesure (24).

10. Dispositif selon la revendication 9, **caractérisé en ce que** devant la cellule de mesure (24) est par ailleurs placé au moins un autre réservoir de stockage (18) servant à recevoir un liquide ou une substance pour l'augmentation du pH de l'échantillon de lait composé de lait écrémé.

11. Dispositif selon l'une des revendications précédentes 6 à 10, **caractérisé en ce que** la cellule de mesure (24) comprend un réservoir de collecte des échantillons (25), une source lumineuse (26) et une cellule photo-électrique (27).

12. Dispositif selon l'une des revendications précédentes 6 à 11, **caractérisé en ce qu'**un équipement d'exploitation et/ou d'affichage (11) est associé à la cellule de mesure (24).

13. Dispositif selon la revendication 12, **caractérisé en ce que** l'équipement d'exploitation et/ou d'affichage (11) est muni d'une échelle graduée qui indique directement la teneur résiduelle en matières grasses de l'échantillon de lait.

14. Dispositif selon l'une des revendications précédentes 6 à 13, **caractérisé en ce qu'**il est configuré comme unité de mesure séparée qui doit être associée à la centrifugeuse.
